# EUROPEAN PATENT SPECIFICATION

(11) **EP 0 192 741 B1**
(45) Date of publication and mention of the grant of the patent: **16.06.1993**
(21) Application number: 85904538.7
(22) Date of filing: 30.08.1985
(51) Int. Cl.: C12N 15/82, C12P 11/00, C12N 1/00

(54) **$i(BACILLUS THURINGIENSIS) CRYSTAL PROTEIN GENE TOXIN SEGMENT**
TOXIN-SEGMENT DES KRISTALLGENS VOM BACILLUS THURINGIENSIS
SEGMENT DE LA TOXINE DU GENE DE LA PROTEINE CRISTALLINE DU $i(BACILLUS THURINGIENSIS)

(30) Priority: 31.08.1984 US 646690
(43) Date of publication of application: 03.09.1986
(73) Proprietor: WASHINGTON RESEARCH FOUNDATION, Seattle, WA 98105 (US)
(72) Inventor: WHITELEY, Helen, Riaboff, Seattle, WA 98125 (US); SCHNEPF, Harry, Ernest, Seattle, WA 98103 (US)
(74) Representative: Bizley, Richard Edward
(86) International application number: US8501665
(87) International publication number: WO8601536

(56) References cited:
- EP-A- 0 063 949
- EP-A- 0 093 062
- THE JOURNAL OF BIOLOGICAL CHEMISTRY, vol. 258, no. 3, 10th February 1983, pages 1960-1967, US; H.C. WONG et al.: "Transcriptional and translational start sites for the Bacillus thuringiensis crystal protein gene"
- PROC. NATL. ACAD. SCI., vol. 79, October 1982, pages 6065-6069; G.A. HELD et al.: "Cloning and localization of the lepidopteran protoxin gene of Bacillus thuringiensis subsp. kurstaki"
- THE JOURNAL OF BIOLOGICAL CHEMISTRY, vol. 260, no. 10, 25th May 1985, pages 6273-6280, The American Society of Biological Chemists, Inc., US; H.E. SCHNEPF et al.: "Delineation of a toxin-encoding segment of a Bacillus thuringiensis crystal protein gene"
- THE JOURNAL OF BIOLOGICAL CHEMISTRY, vol. 260, no. 10, 25th May 1985, pages 6264-6272, US; H.E. SCHNEPF et al.: "The amino acid sequence of a crystal protein from Bacillus thuringiensis deduced from the DNA base sequence"
- PROC. NATL. ACAD. Sci., vol. 78, May 1981, SCHNEPF et al. "Cloning and expression of the Bacillus thuringiensis Crystal protein gene in E. coli" pp. 2893-97
- GENE, vol. 19, 1982, VIEIRA et al."The pUC plasmids, an M13 mp7-derived system for insertion mutagenesis and sequencing with synthetic universal primers" pp. 259-268

## Description

### Field of the Invention

This invention relates generally to DNA sequences capable of being expressed in genetically engineered host organisms. More particularly, this invention relates to an expressible DNA fragment coding for the toxin portion of Bacillus thuringiensis crystal protein.

### Background of the Invention

As is well known, Bacillus thuringiensis crystal protein is toxic to the larvae of a number of lepidopteran insects. As a result preparations containing Bacillus thuringiensis crystals are used commercially as a highly selective biological insecticide. Unfortunately, relatively high manufacturing costs and problems connected with the use of the crystals have made it difficult for such insecticides to compete effectively with other commercially available products.

Wild-type Bacillus thuringiensis produce crystal protein only during sporulation. Such a growth phase limitation, particularly in an industrial process, can result in inconvenience and excessive time requirements during manufacture. This of course increases the costs of the final crystal protein insecticide product.

To overcome the growth phase limitations of wild-type Bacillus thuringiensis, U.S. Patents 4,448,885 and 4,467,036, issued May 15, 1984 and August 21, 1984, respectively, to Schnepf and Whiteley, disclose expression of Bacillus thuringiensis crystal protein by use of novel recombinant plasmids containing expressible heterologous DNA coding for crystal protein protoxin polypeptide. The Schnepf/Whiteley patents also disclose that genetically engineered bacterial host strains, transformed by the novel recombinant plasmids, express Bacillus thuringiensis crystal protein protoxin polypeptide. Such genetically engineered bacterial host strains express Bacillus thuringiensis crystal protein protoxin polypeptide at all stages of growth.

It is now known that in the Bacillus thuringiensis subspecies that synthesize lepidopteran toxins, the crystal protein crystal is composed of one or more protoxin polypeptides of Mr=135,000 to 160,000 (Calabrese, et al., 1980; Tyrell, et al., 1981). Upon dissolution and proteolytic degradation of crystals in vitro and presumably also in the insect midgut, each protoxin molecule yields a toxin peptide of Mr=55,000-72,000 (Bulla, et al., 1979; Lilley, et al., 1980; Chestukhina, et al., 1982).

Since the crystal protein protoxin polypeptides are approximately twice the size of the toxic fragments, insecticidal preparations containing the protoxin crystals could be made to be twice as effective per given dose (or equally as effective at half the dose) if they contained the smaller toxin fragment rather than the larger protoxin polypeptide. In addition, if genetically engineered host strains could be transformed to express the toxin fragment rather than the protoxin polypeptide, the output of the host could be increased and possibly even doubled. To that end it would be useful to identify the specific segment of a Bacillus thuringiensis crystal protein gene that codes for the toxin fragment. It would also be useful to demonstrate that such a DNA segment is expressible in host organisms and that the resultant protein product is toxic to lepidopteran insects.

### Objects

It is an object of the present invention to identify the segment of a Bacillus thuringiensis crystal protein gene, referred to herein as the "toxin-encoding" segment, which codes for the portion of a crystal protein that is toxic to lepidopteran insects.

It is a further object of the present invention to determine the DNA sequence of the "toxin-encoding" segment of a Bacillus thuringiensis crystal protein gene.

It is a further object of the present invention to demonstrate that a Bacillus thuringiensis crystal protein "toxin-encoding" gene segment is expressible in transformed recombinant host organisms.

It is a further object of the present invention to demonstrate that the protein product produced by recombinant hosts transformed to express a Bacillus thuringiensis crystal protein "toxin-encoding" gene segment is toxic to lepidopteran insects.

Other objects of the present invention will become apparent to those skilled in the art from the following description and figures.

### FIGURES

### General Description of the Figures

FIGURE 1 (views A and B) shows restriction enzyme maps and sequencing strategy for a Bacillus thuringiensis crystal protein gene;
FIGURE 2 (views A and B) shows the DNA sequence of a Bacillus thuringiensis crystal protein gene;
FIGURE 3 (views A and B) shows restriction maps of pES1 and construction of pHES16;
FIGURE 4 (views A-E) shows the construction strategy for the 3'-end deletions of a crystal protein gene in pHES16;
FIGURE 5 (views A-C) shows construction of plasmids fusing a crystal protein gene to the lac promoter;
FIGURE 6 (views A-C) shows the 3'-end deletions of a crystal protein gene; and
FIGURE 7 (views A-C) shows transcriptional and translational fusions of lacZ to the crystal protein gene and to crystal protein genes modified at the 3' and/or 5' ends.

### Detailed Description of the Figures

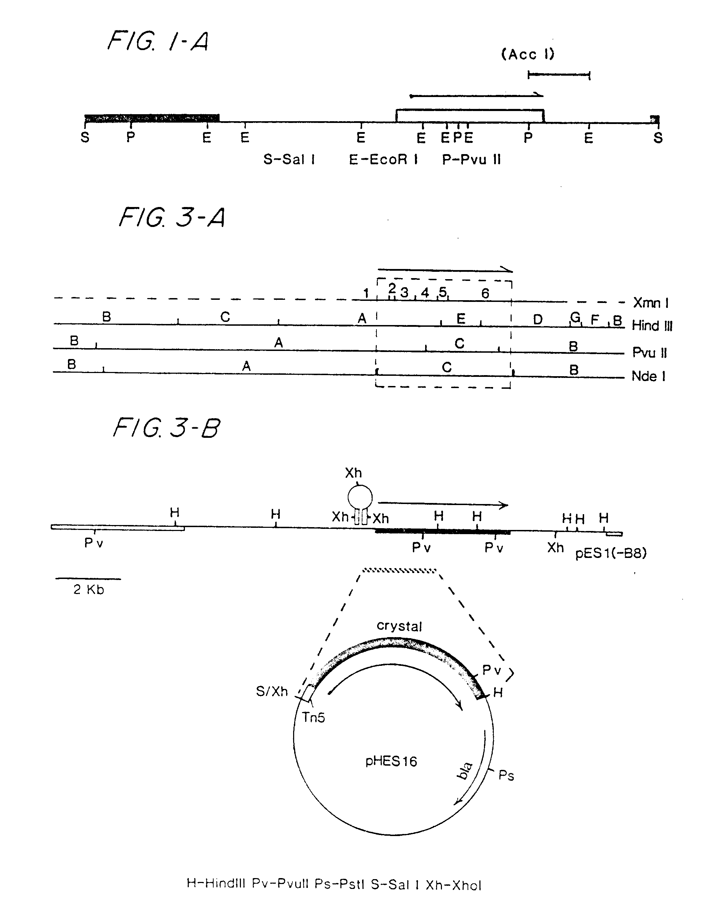

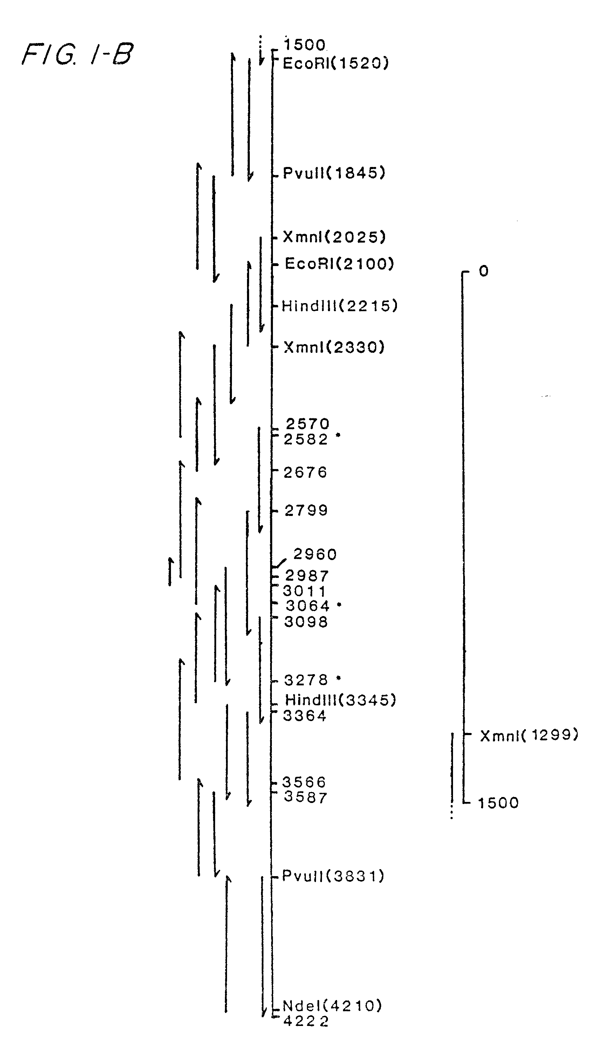

FIGURE 1A is a restriction enzyme map of pES1 showing the portion of the plasmid which was sequenced (boxed segment), the length and direction of the crystal protein transcript (arrow), and the portion of a subcloned restriction fragment which was used as an S1 nuclease mapping probe (bracketed line). The thicker lines represent vector (pBR322) sequences.

FIGURE 1B is a restriction enzyme map and sequencing strategy for ca. 2800 bp of the crystal protein gene. The sequence of the first 1500 bp has been reported (Wong, et al., 1983). Arrows indicate the length and direction of sequence determination from the sites presented.

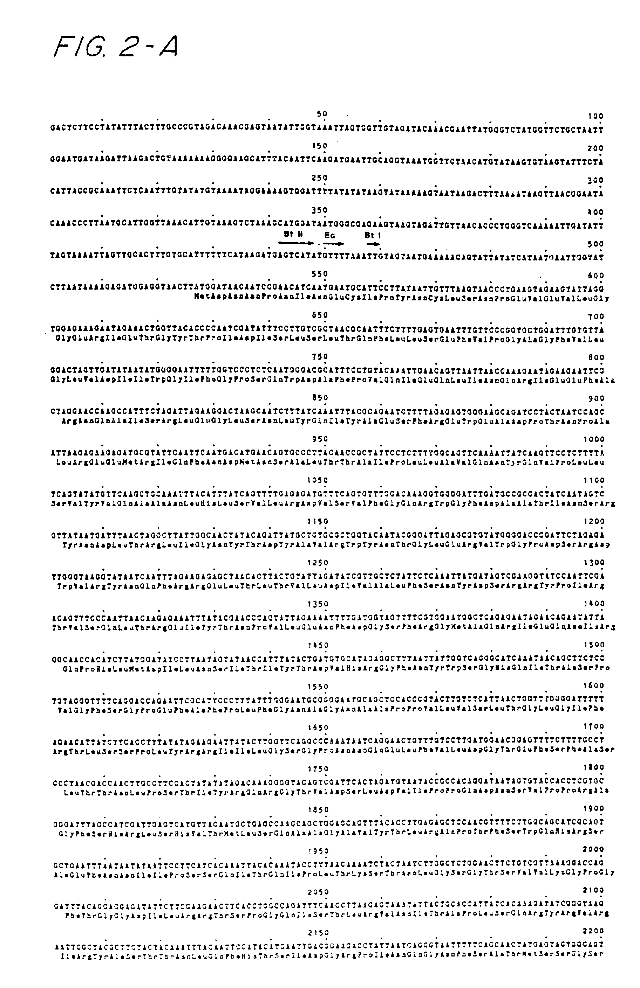

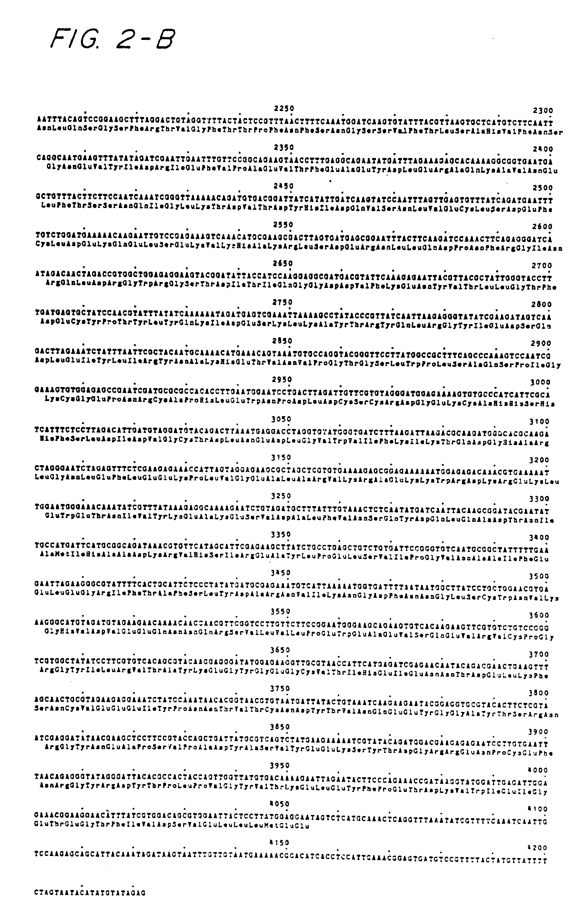

FIGURES 2A and 2B show the DNA sequence of the crystal protein gene, including some 5' and 3' flanking sequences. The start sites of transcription in Bacillus thuringiensis (BtI and BtII) and in Escherichia coli (Ec) are indicated as well as the entire deduced amino acid sequence. FIGURE 2A includes nucleotides 1 through 2200, plus corresponding codons 1 through 558. FIGURE 2B includes nucleotides 2201 through 4222, plus codons 559 through 1176. Nucleotides 4053 through 4055 comprise codon 1176 which codes for the carboxy terminal amino acid in the Bacillus thuringiensis crystal protein. Nucleotides 4140 through 4185 comprise the transcriptional terminator for this gene.

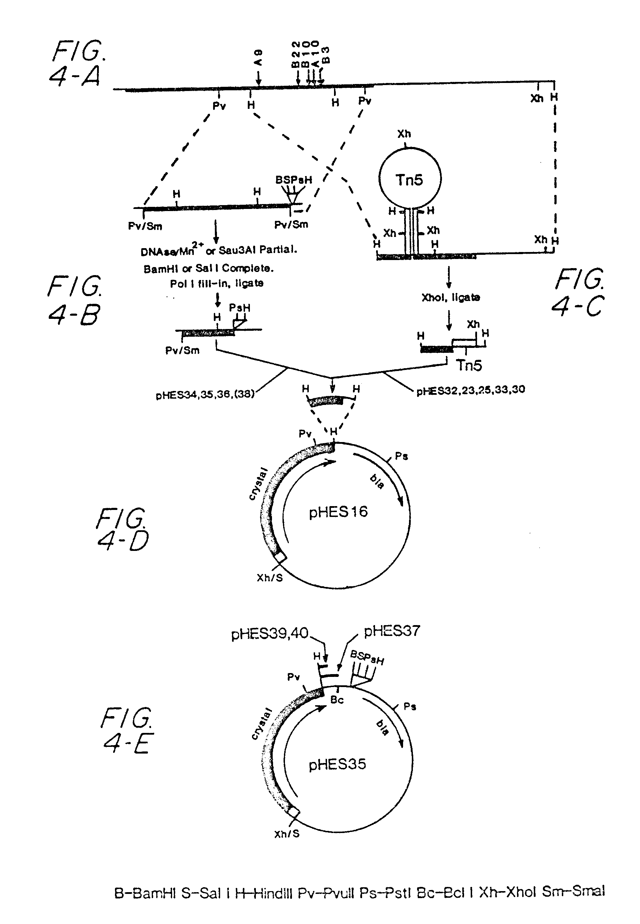

FIGURE 3A shows restriction enzyme site maps of pES1 linearized at the single SalI site. A partial map is shown for XmnI (solid line) and complete maps are shown for HindIII, PvuII, and HdeI. The location of the crystal protein gene is shown with a dashed box, and an arrow indicates the direction of transcription.

FIGURE 3B is a restriction enzyme map of pES1 which shows the location of Tn5 insertion B8 (stem-and-loop structure), the position of the crystal protein gene (thickened line), the direction of transcription (arrow), and the position of pBR322 vector sequences (boxed lines). The XhoI-HindIII fragment used to construct pHES16 (cross-hatched line) and its position in that plasmid are indicated.

FIGURE 4A is an expanded restriction enzyme map of a segment of pES1 containing the crystal protein gene (thickened line) and 3' flanking sequences. The positions of Tn5 insertions in the HindIII E fragment are indicated with arrows.

FIGURE 4B shows the strategy for obtaining 3'-proximal deletions in the HindIII E fragment of pES1 from bacteriophage M13mp8 containing the PvuII C fragment of pES1 cloned into the SmaI site in the indicated orientation.

FIGURE 4C shows the strategy for constructing deletions in pES1 containing Tn5 insertions in the HindIII E fragment.

FIGURE 4D illustrates the method used to extend the crystal protein gene segment in pHES16 to deletion endpoints in the HindIII E fragment of pES1. The plasmids derived from the M13 phages or the Tn5 insertion mutants are indicated on the connecting arrows. (pHES38, indicated parenthetically, was made in the manner indicated from a phage containing XmnI fragment 5 (FIGURE 3A) and required no further deletion.

FIGURE 4E illustrates the strategy used to make deletion derivatives (pHES37, 39, 40) of pHES35. (See FIGURE 4D for the construction of pEHS35.)

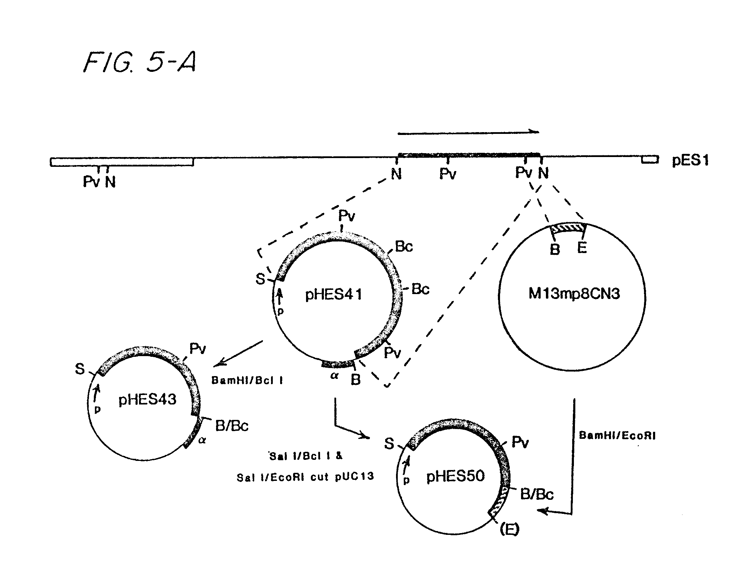

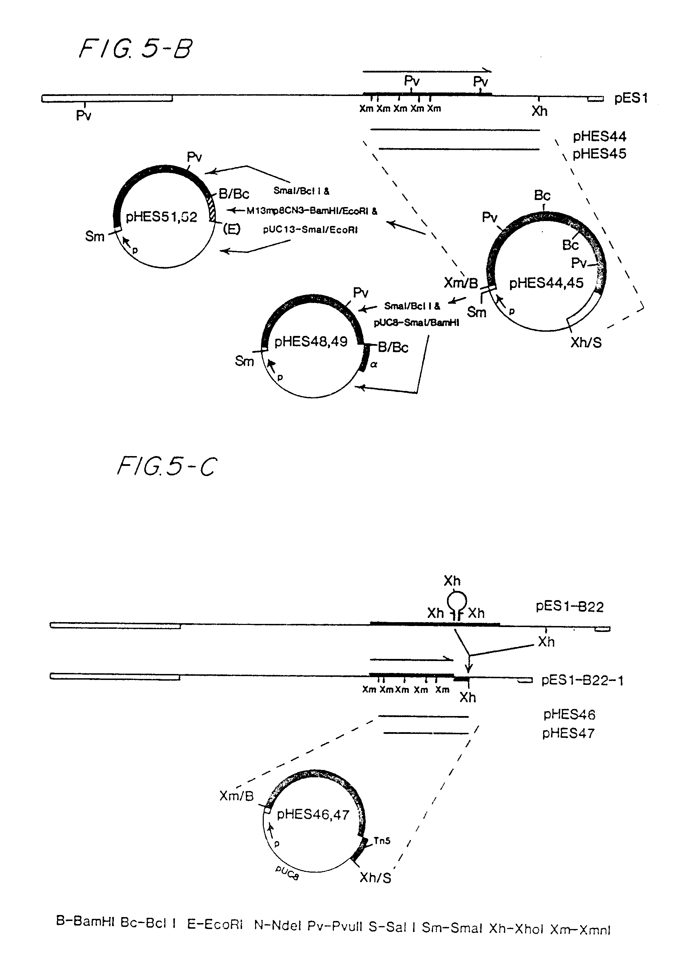

FIGURE 5A shows transcriptional fusion of the lac promoter to the crystal protein gene. A map of pES1 linearized by SalI is presented, showing the location (thickened line) and direction of transcription (arrow) of the crystal protein gene. Vector sequences are boxed. The location and direction of transcription of the lac promoter are indicated with a P and an arrow. The location of the lac alpha-complementation segment is indicated by the symbol for the Greek letter alpha.

FIGURE 5B shows translational fusion of the lacZ gene to the 10th and 50th codons of the crystal protein gene. A map of pES1 indicating the position and orientation of the crystal protein gene is presented. The XmnI partial/XhoI complete digest fragments used in pHES 44 and 45 are also indicated by lines. A detailed description of these constructions is found in the Materials and Methods section, supra.

FIGURE 5C shows translational fusions of the lacZ gene to the 10th and 50th codons of a truncated Tn5 insertion mutation of the crystal protein gene. Maps of pES1-B22 and pES1-B22-1, its XhoI deletion derivative, are shown. The XmnI partial/XhoI complete digestion fragments used to make pHES46 and 47 are indicated with lines beneath the maps.

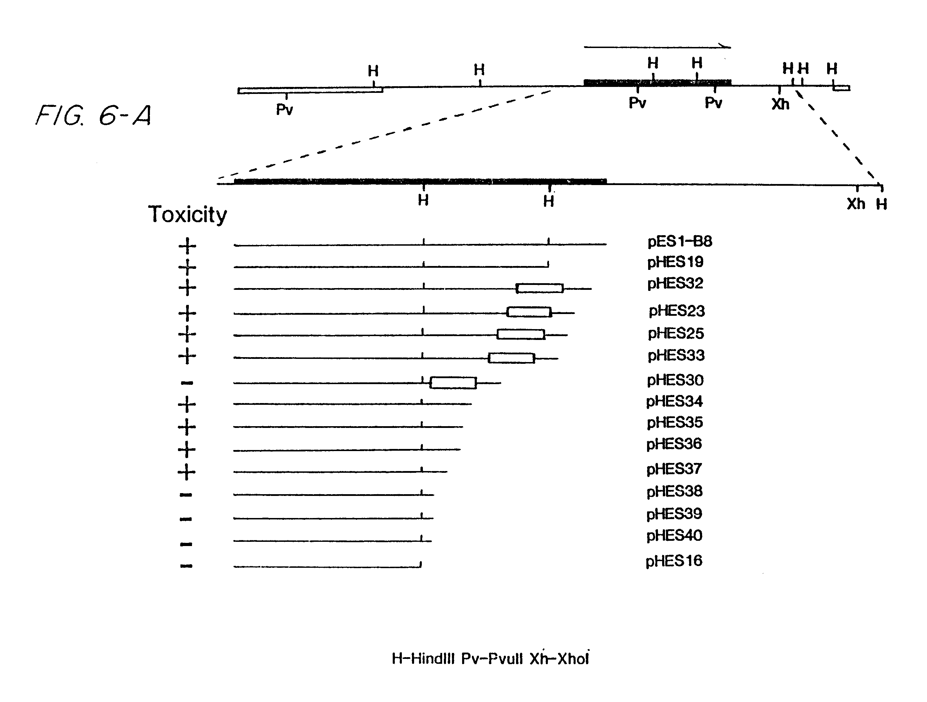

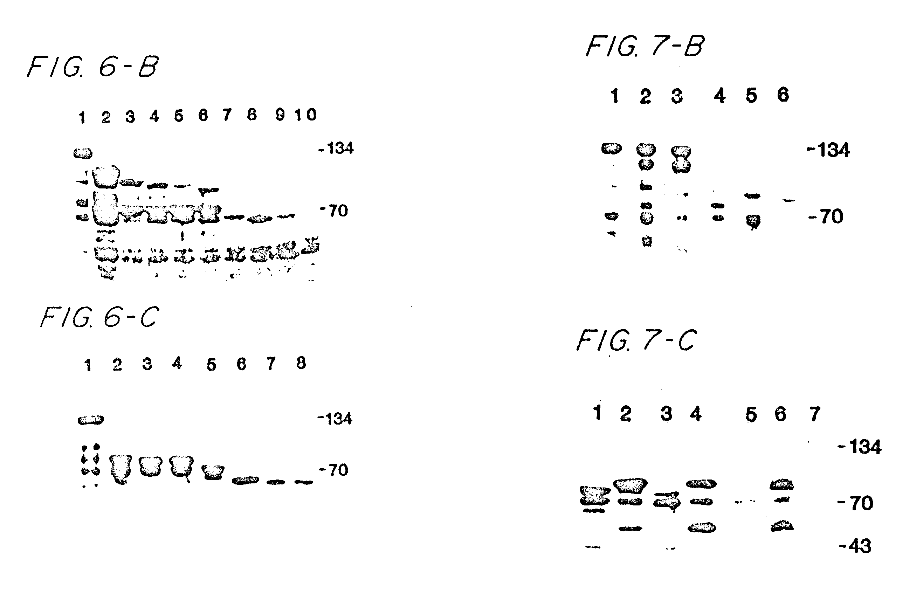

FIGURE 6A is a restriction enzyme map of pES1 with an expanded map of the crystal protein gene region. The crystal protein gene (thickened line), its direction of transcription (arrow) and pBR322 vector sequences (boxed lines) are indicated. The portion of the crystal protein gene remaining in the indicated plasmids is shown by a line under the restriction enzyme maps. Boxed segments of these lines indicate Tn5 sequences. The results of toxicity test for Escherichia coli containing these plasmids are also shown.

FIGURE 6B shows immunoblots of extracts of Escherichia coli carrying the following plasmids: lanes 1, pES1-B8; 2, pHES19; 3, pHES32; 4, pHES23; 5, pHES25; 6, pHES33; 7, pHES30; 8, pHES16; 9 pHES31 (containing the HindIII insert of pHES32 in reversed orientation); 10, pHES322. Lane 1 contains 1 microliter of extract; lanes 2-10 contain 20 microliters of extract.

FIGURE 6C shows immunoblots of extracts of Escherichia coli carrying the following plasmids: lanes 1, pES1-B8; 2, pHES34; 3, pHES35; 4, pHES36; 5, pHES37; 6, pHES38; 7, pHES39; 8, pHES40. Lane 1 contains 1 microliter of extract; lanes 2-8 contain 20 microliters of extract.

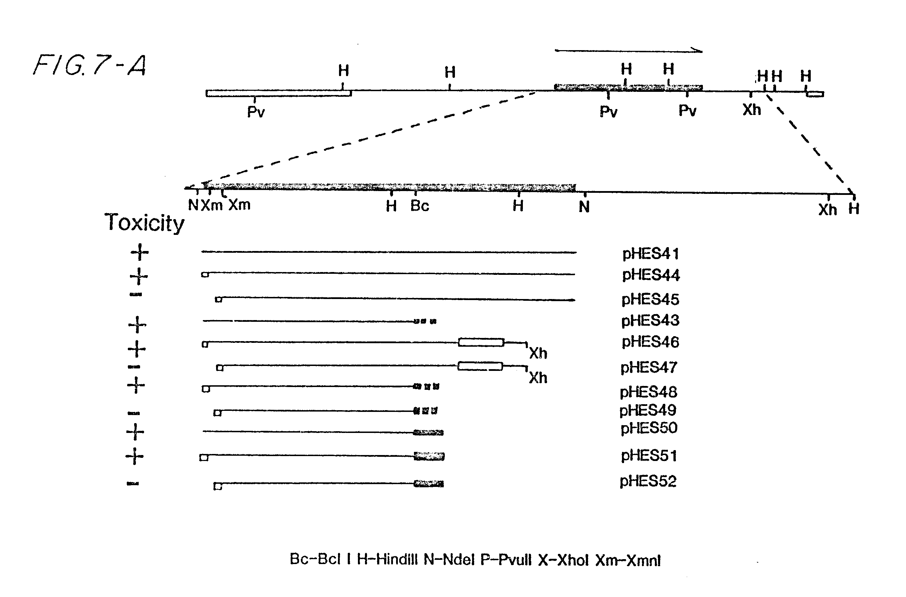

FIGURE 7A is a restriction enzyme map of pES1 with an expanded map of the crystal protein gene region. The crystal protein gene (thickened line), its direction of transcription (arrow) and pBR322 vector sequences (boxed lines) are indicated. Lines below the maps showing the amount of crystal protein gene remaining in the indicated plasmids are shown under the restriction enzyme maps. For the deleted plasmids, boxes at the left end indicate translational fusions to lacZ; boxes at the right end indicate Tn5 sequences. Dashed thickened lines at the right end indicate lac alpha-peptide sequences and the non-dashed thickened lines at the right end indicate the final 74 codons and transcriptional terminator of the crystal protein gene. The results of toxicity tests for Escherichia coli containing these plasmids are also shown.

FIGURE 7B shows immunoblots of Escherichia coli carrying the following plasmids: Lanes 1, pHES41; 2, pHES44; 3, pHES45; 4, pHES43; 5, pHES46; 6, pHES47. Lanes 1-3 contained 5 microliters of extract; lanes 4-6 contained 10 microliters of extract.

FIGURE 7C shows immunoblots of Escherichia coli carrying the following plasmids: Lanes 1, pHES43; 2, pHES50; 3, pHES48; 5, pHES49; 6, pHES52; 7 pBRS322. Each lane contained 20 microliters of extract.

### References

The present disclosure cites the following publications:
1. Auerswald, E.-A., Ludwig, G. and Schaller, H. (1981) Cold Spring Harbor Symp. Quant. Biol., 45, 107-114.
2. Berg, D.E. and Berg, C.M. (1983), Biotechnol., 1, 417-435.
3. Birnboim, H.C. and Doly, J. (1979), Nucl. Acids Res., 7, 1513-1523.
4. Bolivar, F., Rodriguez, R.L., Greene, P.S., Betlach, M.C., Heynecker, H.L., Boyer, H.W., Crosa, J.H. and Falkow, S. (1977), Gene, 2, 95-113.
5. Bulla, L.A., Jr., Davidson, L.I., Kramer, K.J. and Jones, B.E. (1979) Biochem. Biophys. Res. Comm. 91, 1123-1130.
6. Bulla, L.A., Jr., Kramer, K.J., Cox, D.J., Jones, B.L., Davidson, L.I. and Lookhart, G.L. (1981), J. Biol. Chem., 256, 3000-3004.
7. Calabrese, D.M., Nickerson, K.W. and Lane, L.C. (1980) Can. J. Microbiol 26, 1006-1010.
8. Chestukhina, G.G., Kostina, L.I., Mikhailova, A.L., Tyurin, S.A., Klepikova, F.S. and Stepanov, V.M. (1982), Arch. Microbiol., 132, 159-162.
9. Court, D., Huang, T.F. and Oppenheim, A.B. (1983), J. Mol. Biol., 166, 233-240.
10. Dulmage, H.T. and Cooperators (1980) in Microbial Control of Pests and Plant Diseases 1970-1980) (Burges, H.D., ed.) pp. 193-222, Academic Press, London.
11. Fowler, A.V. and Zabin, I. (1983), J. Biol. Chem., 258, 14354-14358.
12. Goldfarb, D.S., Rodriguez, R.L. and Doi, R.H. (1982), Proc. Nat. Acad. Sci. USA, 79, 5886-5890.
13. Gray, O. and Chang, S. (1981), J. Bacteriol., 145, 422-428.
14. Held, G.A., Bulla, L.A., Ferrari, E., Hoch, J., Aronson, A.I. and Minnich, S.A. (1982), Proc. Natl. Acad. Sci. USA, 79, 6065-6069.
15. Holmes, W.M., Platt, T. and Rosenberg, M. (1983), Cell, 32, 1029-1032.
16. Hong, G.F. (1982), J. Mol. Biol., 158, 539-549.
17. Huber, H.E., and Luthy, P. (1981) in Pathogenesis of Invertebrate Microbial Diseases (Davidson, E.W., ed.) pp. 209-234, Allanheld, Osmun and Co., Totowa, NJ.
18. Huber, H.E., Luthy, P., Ebersold, H.R. and Cordier, J.-L. (1981a) Arch. Microbiol., 129, 14-18.
19. Klier, A., Fargette, F., Ribier, J. and Rapoport, G. (1982), EMBO J., 7, 791-799.
20. Klier, A., Lecadet, M.-M. and Rapoport, G. (1978), In Spores VII (Chambliss, G. and Vary, J.C., eds.), pp. 205-212, American Society for Microbiology, Washington, D.C.
21. Klier, L.A., Parsot, C. and Rapoport, G. (1983), Nucl. Acids Res., 11, 3973-3987.
22. Kronstad, J.W., Schnepf, H.E. and Whiteley, H.R. (1983), J. Bacteriol., 154, 419-428.
23. Lilley, M., Ruffell, R.N. and Somerville, H.J. (1980), J. Gen. Microbiol., 118, 1-11.
24. Mandel, M. and Higa, A. (1970), J. Mol. Biol., 53, 159-162.
25. Maniatis, T., Fritsch, E.F. and Sambrook, J. (1982) in Molecular Cloning, Q Laboratory Manual, Cold Spring Harbor.
26. Messing, J., Crea, R. and Seeburg, P.H. (1981), Nucl. Acids Res., 9, 309-321.
27. Messing, J. and Vieira, J. (1982), Gene, 19, 269-276.
28. Nicoll, D. Ph.D. Thesis, University of Washington, Seattle, Washington (1983).
29. Petit-Glatron, M.-F. and Rapoport, G. (1975) in Spores VI (Gerhardt, P., Costilow, R.N. and Sadoff, H.L., eds.), pp. 255-264, American Society for Microbiology, Washington, D.C.
30. Polisky, B.,. Greene, P., Garfin, D.E., McCarthy, B.J., Goodman, H.M. and Boyer, H.W. (1975) Proc. Nat. Acad. Sci. USA, 72, 3310-3314.
31. Sanger, F., Nicklen, S. and Coulson, A.R. (1977), Proc. Natl. Acad. Sci. USA, 74, 5463-5467.
32. Schesser, J.H., Kramer, K.J. and Bulla, L.A., Jr. (1977) Appl. Environ. Microbiol., 33, 878-880.
33. Schnepf, H.E. and Whiteley, H.R. (1981), Proc. Nat. Acad. Sci. USA, 78, 2893-2897.
34. Sutcliff, J.G. (1979) Cold Spring Harbor Symp. Quant. Biol., 43, 77-90.
35. Traboni, C., Cortese, R., Ciliberto, G. and Cesareni, G. (1983), Nucl. Acids Res., 11, 4229-4239.
36. Tyrell, D.J., Bulla, L.A., Andrews, R.E., Kramer, K.J., Davidson, L.I. and Nordin, P. (1981), J. Bacteriol., 145, 1051-1062.
37. Vieira, J. and Messing, J., (1982), Gene, 19, 259-268.
38. von Gabain, A., Belasco, J.G., Schottel, J.L., Chang, A.C.Y. and Cohen, S.N. (1983), Proc. Nat. Acad. Sci. USA, 80, 653-657.
39. Whiteley, H.R., Kronstad, J.W., Schnepf, H.E. and DesRosier, J.P., in Molecular Cloning and Gene Regulation in Bacilli (A.T. Ganesan, S. Chang and J.A. Hoch, editors) Academic Press, New YOrk (1982) at pp. 131-144.
40. Wong, H.C., Schnepf, H.E. and Whiteley, H.R. (1983), J. Biol. Chem., 258, 1960-1967.

### Definitions

In the present description and claims reference will be made to terms and phrases which are expressly defined for use herein as follows:

As used herein, the letters A, T, C, G are meant to denote the nucleotides adenine, thymine, cytosine and guanine in DNA, respectively.

As used herein, bp means base pairs.

As used herein, substantial sequence homology is meant to denote nucleotide sequences that are substantially functionally equivalent to one another. Nucleotide differences between such sequences having substantial sequence homology will be de minimus in affecting the function of the gene products coded for by such sequences.

As used herein, amino acid abbreviations are:

| | | | |
|---|---|---|---|
| Phenylalanine | Phe | Histidine | His |
| Leucine | Leu | Glutamine | Gln |
| Isoleucine | Ile | Asparagine | Asn |
| Methionine | Met | Lysine | Lys |
| Valine | Val | Aspartic acid | Asp |
| Serine | Ser | Glutamic acid | Glu |
| Proline | Pro | Cysteine | Cys |
| Threonine | Thr | Tryptophan | Try |
| Alanine | Ala | Arginine | Arg |
| Tyrosine | Tyr | Glycine | Gly |

As used herein, crystal protein gene means a DNA segment that codes for a Bacillus thuringiensis crystal protein peptide that is toxic to lepidopteran insects. The term "protoxin" means a crystal protein polypeptide of ca. Mᵣ=130,000 - 160,000 which upon dissolution and proteolytic degradation (in vitro and presumably also in the insect midgut) yields a smaller "toxin" fragment of ca. Mᵣ 55,000-73,000 that is itself toxic to lepidopteran insects. The phrase "amino terminal 55% of the Bacillus thuringiensis crystal protein gene" means the amino terminal 645 codons of the crystal protein gene as shown in FIGURES 2A and 2B.

As used herein, transcriptional terminator means that DNA sequence which promotes cessation of transcription. When the term is used to describe the transcriptional terminator sequence for the Bacillus thuringiensis crystal protein gene shown in FIGURES 2A and 2B, the term is meant to encompass the sequence comprised of base pairs 4140 through 4185. (See FIGURE 2B).

As used herein, the phrase "final 74 codons" means the final 74 codons of the Bacillus thuringiensis crystal protein gene as shown in FIGURES 2A and 2B. More specifically, the final 74 codons are comprised of codons 1103 through 1176 (or base pairs 3833 through 4054) as shown in FIGURE 2B.

The methods of the present invention make use of techniques of genetic engineering and molecular cloning. As used herein, genetic engineering means techniques which lead to the formation of new combinations of heritable material by the insertion of nucleic acid molecules, produced or derived by whatever means outside the cell, into a bacterial plasmid or other vector system so as to allow their incorporation into a host organism in which they do not naturally occur but in which they are capable of replication. Host organisms carrying these new combinations of heritable material are referred to herein as recombinant host organisms. General techniques of genetic engineering and molecular cloning are included in Maniatis, et al., Molecular Cloning: A Laboratory Manual, Cold Spring Harbor Laboratory (1982).

### Description of the Invention

### Summary of the Invention

The present invention comprises a DNA fragment that codes for the portion of Bacillus thuringiensis crystal protein peptide that is toxic to lepidopteran insects. The invention also comprises the DNA and amino acid sequences for the disclosed toxin-encoding DNA fragment. In addition the invention demonstrates that the disclosed toxin-encoding DNA fragment (referred to herein as the Bacillus thuringiensis crystal protein gene toxin segment) is expressible in recombinant host organisms, and that the "toxin" protein product produced by the transformed hosts is toxic to lepidopteran insects.

### Detailed Description of the Invention

It is known that many subspecies of Bacillus thuringiensis produce crystal proteins that are toxic to lepidopteran insects. Is is also known that these crystal proteins are coded for by crystal protein genes (Schnepf and Whiteley, 1981; Kronstad, et al., 1983; Held, et al., 1982) which, depending on the subspecies, may be located on large plasmids and/or the chromosome (Kronstad, et al., 1983; Held, et al., 1982; Klier, et al., 1982; Schnepf and Whiteley, 1981).

We have cloned a crystal protein gene from Bacillus thuringiensis subspecies kurstaki HD-l-Dipel and have shown that the gene is located on a large plasmid in this strain. See U.S. Patent 4,467,036. In addition we have shown that the crystal protein gene from subspecies kurstaki HD-l-Dipel is homologous to crystal protein genes on one or more plasmids (and in one case the chromosome of) several Bacillus thuringiensis strains. See U.S. Patent 4,467,036; also see Kronstad, et al. (1983).

In a previous communication the transcriptional and translational start sites and the nucleotide sequence for approximately one-fourth of the gene cloned from Bacillus thuringiensis subspecies Kurstaki HD-l-Dipel were reported (Wong, et al., 1983). However, since more than this partial sequence was required to determine which portion of the crystal protein gene coded for the "toxin" portion of the crystal protein, we determined the DNA sequence for the remainder of the gene.

Our sequencing strategy is shown in FIGURE 1; also see the Materials and Methods section, supra. The DNA sequence is shown in FIGURES 2A and 2B as is the amino acid sequence deduced from the only open reading frame in the DNA sequence. This sequence contains the NH₂-terminal sequence determined previously (Wong, et al., 1983) by chemical methods.

The disclosed DNA sequence codes for 1176 amino acids specifying a polypeptide with a calculated molecular weight of 133,500. As discussed in Example I, this value for the crystal protein protoxin peptide agrees well with molecular weights determined for the protoxins from Bacillus thuringiensis subsp. kurstaki and subsp. thuringiensis. In addition the deduced amino acid composition is very similar to the chemically determined amino acid compositions of either whole crystals or the purified protoxin of Bacillus thuringiensis subsp. kurstaki (See Table 1, supra.)

Knowing that the Bacillus thuringiensis crystal protein protoxin can be cleaved to yield a smaller toxin fragment, we created recombinant Escherichia coli strains bearing deletions and fusions of the crystal protein gene and then analyzed the proteins they produced to delineate the portion of the gene which encodes the toxin peptide. Construction of these "deletion and fusion" plasmids is discussed in the Materials and Methods section, supra; also see FIGURES 3-7. The truncated peptides produced by recombinant hosts transformed by these plasmids are discussed in Examples II through V.

Very generally, the truncated peptides produced by host strains transformed by the "deletion and fusion" plasmids indicate that the amino terminal 55% of the crystal protein gene encodes sufficient information to produce a lepidopteran toxin. More specifically, the "deletion and fusion" results indicate that deletions to the 50th codon from the 5' end of the gene, or to the 603rd codon from the 3' end abolish toxicity, while deletions to the 10th codon from the 5' end, or to the 645th codon from the 3' end do not. They also indicate that the 3' end of the crystal protein gene, from codons 645 to 1176 is not essential for toxicity, and the first 10 codons can be replaced by two different segments containing the N-terminus of beta-galactosidase and synthetic linker sequences without eliminating toxicity.

The shortest toxic segment of the crystal protein gene reported here encodes a polypeptide of ca. Mᵣ 73,000; the outermost deletion endpoints for non-toxic mutants (codons 50 through 603) would encode a peptide of ca. Mᵣ 63,000. Others have reported toxic proteolytic fragments of the crystal protein in the Mᵣ 30,000 to 80,000 range (Huber and Luthy, 1981), with the most recent reports indicating a size of Mᵣ 55,000 to 72,000 (Bulla, et al., 1979; Lilley, et al., 1980 and Chestukhina, et al., 1982).

While differences in molecular weight standards, electrophoresis systems and Bacillus thuringiensis strains could account for the differences between our current results and those of investigators reporting toxic fragments of Mᵣ 67,000 to 72,000, it is also possible that smaller toxic fragments could come from a shorter segment of the gene than that delineated in the present investigation. We point out that the major difference between our study and the preceding work is that proteases were used in the earlier studies to generate smaller toxic peptides from solubilized preparations of crystals whereas we have used Escherichia coli cells to synthesize toxic proteins from altered genes. Assuming that the shorter toxic peptides are encoded by the segment of the crystal protein gene delineated here, our finding that shorter segments of the gene produced non-toxic peptides may be related to this difference in methodology. It is possible that deletions into the minimum toxic segment of the gene remove amino acid segments which are either necessary for attainment of the toxic conformation, or are required to prevent "non-toxin" portions of the remaining polypeptide segment from blocking attainment of the toxic conformation of the protein.

The results of this study are, however, in broad agreement with previous work showing that the N-terminus of the crystal protein was present on a toxic polypeptide fragment (Chestukhina, et al., 1982). In addition, our experiments involving 3'-end deletions or gene fusions indicate that there is a segment of increased susceptibility to proteolysis between residue 603 and 645 which leads to the production of an N-terminal ca. Mᵣ 70,000 fragment in the deleted strains. The location of this site near residue 645 is inferred from the results obtained with successive 3'-end deletions. More specifically, a plasmid in which crystal protein sequences terminate at codon 645 directs synthesis of little, if any, polypeptide beyond the cleavage site. The presence of a processing site before residue 645 is implied by apparent removal of the lac alpha-peptide in extracts of Escherichia coli containing a plasmid having the lac alpha-peptide sequences fused in phase following codon 645 of the crystal protein gene. The lac alpha-peptide is functional in this plasmid, since it complements the beta-galactosidase activity in Escherichia coli JM83 or JM103 (data not shown).

The different N-terminal amino acids reported by several investigators for the toxic fragments of the crystal protein (Lilley, et al., 1980; Chestukhina, et al., 1982) may reflect some flexibility in the N-termini which can be present on these fragments. This is supported by our current study which indicates that the first 10 codons of this gene can be replaced by two different polypeptide encoding segments without eliminating toxicity.

The 5'-end and 3'-end alterations of the crystal protein gene reported herein involved fusing these sequences to new sequences, some of which had unusual effects on the expression of the altered proteins in Escherichia coli. When the entire crystal protein gene was present, the 5'-end fusions to beta-galactosidase at the 10th and 50th codons of the crystal protein gene resulted in the production of a ca. Mᵣ 110,000 antigen in addition to the Mᵣ 134,000 crystal protein. This may indicate an altered conformation that is more sensitive to degradation. In addition, the 50th codon fusion showed little or no processing to a ca. Mᵣ 70,000 fragment (and no toxicity) when two different 3'-end deletions were present. However, some Mᵣ 70,000 material was detected when this altered gene had the final 74 codons of the crystal protein gene following codon 645. This implies that loss of processing and toxicity may be due to a conformational change mediated by the alteration at the 5' end of the gene. The 50th codon fusion disrupts the most extensive hydrophobic segment (data not shown) of the crystal protein. The lack of toxicity of the bacteria containing this alteration at the 5' end implies that this segment of the protein is either directly involved in toxicity, or that the alteration of this segment prevents the attainment of the toxic conformation of the protein.

In the case of the 10th codon fusion, segments of 9 amino acids or 19 amino acids can be substituted without eliminating toxicity. Preliminary evidence from titration experiments indicates that the protein having the 9 amino acid-substituted 10th codon fusion and the protein containing the natural N-terminus differ in toxicity by less than about 3 fold, if they differ at all. N-terminal substitutions have been reported for other proteins as well, most notably beta-galactosidase (Fowler and Zabin, 1983), the lac alpha-peptide (Vieira and Messing, 1982; Messing and Vieira, 1982; Traboni, et al., 1983; see also above) and chloramphenicol acetyl transferase (Goldfarb, et al., 1982). In beta-galactosidase (Fowler and Zabin, 1983), increased susceptibility to heat or denaturation by urea were reported to be due to N-terminal substitutions.

Fusion of the final 74 codons of the crystal protein gene (codons 1103-1176 in FIGURE 2B), a segment distal to the toxic portion of the molecule, to codon 645 also has an apparent effect on conformation. Although a cleavage product of ca. Mᵣ 70,000 is produced, a much more prominent cleavage product of ca. Mᵣ 50,000 is also detected. This may, however, be an exceptional case since the final 74 residues of the crystal protein might be able to form a domain which can recognize and bind to a site in the amino-terminal region of the protein. If this binding were to take place without the intervening polypeptide sequence, the overall conformation of the protein might be altered, resulting in an increased susceptibility to proteolysis.

In assessing the effects of different 3' ends on gene expression a surprising result was the observation that, given an equivalent promoter-ribosome binding site configuration, the source of the sequence following codon 645 of the crystal protein gene influenced the extent of synthesis of the altered crystal protein. Since we have been unable to detect differences in the stability of these polypeptides, this difference in expression may reflect differences in the stability of mRNAs, although some unusual effect on translation elongation or termination cannot be ruled out. Largely as a result of the study of the regulation of the bacteriophage lambda int gene, where gene expression is regulated, in part, by alternative RNA structures near a transcription termination site (Court, et al., 1983), it has been proposed that transcriptional terminators have a more general role in regulating gene expression by affecting the stability of mRNA (Holmes, et al., 1983).

The termination site used by the altered crystal protein genes which end in pBR322 sequences is most likely distal to the beta-lactamase gene, and consists of three stem-and-loop structures. The mRNA of the beta-lactamase gene has a half-life of ca. 3 min. (von Gabain, et al., 1983). The site at which transcription would terminate when reading into Tn5 is not known, however it is known that Tn5 is highly polar to transcription (Berg and Berg, 1983). On the basis of the result described here, we raise the possibility that there is a transcriptional terminator in the first 485 bp of Tn5.

The crystal protein gene terminator has a very favorable stem-and-loop structure, delta G=-30.4 Kcal/mole (data not shown), and appears to allow the highest level of expression. Possibly, the stability of this structure is related to the reported longer half-life of the crystal protein mRNA (Petit-Glatron and Rapoport, 1975) and the larger amount of toxic peptide detected in strains carrying the terminator at the 3'-end.

Details of these and other specific embodiments of the present invention are outlined in the following examples. Such examples are for illustrative purposes only and are not intended to limit the scope of the claims in any way. The materials and methods utilized in these examples are listed below.

### Materials and Methods Bacterial strains, plasmids and phages.

Plasmid pES1 (see U.S. Patent 4,467,036 issued August 21, 1984; also see Schnepf and Whiteley, (1981)) in Escherichia coli strain HB101 was the source of the crystal protein gene DNA. M13 bacteriophage strains mp7, mp8, and mp9 were propagated on Escherichia coli strain JM103 (Messing, et al., 1981; Messing and Vieira, 1982). Escherichia coli strains CS412 (Gray and Chang, 1981), JM83 and JM103 (Vieira and Messing, 1982), plasmids pBR322 (Bolivar, et al., 1977), pUC8Ap (Vieira and Messing, 1982), pUC13Cm and phage M13mp8 (Messing and Vieira, 1982) are described in the references cited for each. Plasmid and double-stranded phage DNAs were prepared as described in Birnboim and Doly (1979). Transfection of phage DNA was according to Mandel and Higa (1970). The methods used for transposon 5 (Tn5) mutagenesis, plus descriptions of some of the Tn5 insertion mutants in pES1 are described in Wong, et al. (1983). The positions of several additional Tn5 insertions in pES1 are shown in FIGURE 4; they were determined as described in Wong, et al. (1983).

### Enzymes and Radiolabeled Compounds

Restriction enzymes, T4 DNA ligase, and S1 nuclease were purchased either from New England Biolabs or Bethesda Research Laboratories. The large fragment of DNA polymerase I and all ³²P-labeled nucleotides were purchased from New England Nuclear. All enzymes were used as recommended by the suppliers.

### DNA Sequencing

The dideoxynucleotide chain termination procedure of Sanger, et al. (1977) employing M13-derived phages as templates was used for DNA sequencing as described in Wong, et al. (1983). Template phages for sequencing the internal PvuII fragment (bases 1845 to 3831) of the crystal protein gene were obtained by the DNAse I deletion method of Hong (1982). Alternatively, in order to bring the sequencing primer adjacent to several of the Sau3Al sites in the PvuII fragment, the DNA was partially digested with Sau3Al and completely digested with BamHI (the latter enzyme cuts only in the polylinker site of M13 mp8 adjacent to the sequencing primer site). Less-than-full size phage DNA was then purified and recircularized with DNA ligase.

The DNA sequence was translated and analyzed by the use of a computer program (Nicoll, 1983) modified for use on the IBM Personal Computer by Dr. D. Nicoll and Dr. J. Champoux (Department of Microbiology and Immunology, University of Washington).

Preparation and manipulation of plasmid and phage DNAs. Standard methods were used for preparing plasmid and phage DNAs and for digesting and modifying these DNAs (Maniatis, et al., 1982). DNAse I was used in the presence of Mn⁺⁺ (Hong, 1982) to obtain deletions in phage or plasmid clones. The EcoRI* activity was obtained as described by Polisky, et al., (1975).

Immunoblotting. Whole cell extracts of Escherichia coli were prepared as follows: 1 ml of culture in L-broth was centrifuged for 30 sec. in a 1.5 ml Eppendorff tube, the supernatant was removed and the pellet was resuspended in 30 microliters of 0.01 M tris, 0.005 M EDTA pH 7.0. Fifty microliters of hot 2X sample buffer (0.1 M tris, pH 6.8, 2% sodium dodecyl sulfate, 2% beta-mercaptoethanol, 20% glycerol and 0.01% bromphenol blue) were added and the tube was plunged into a boiling water bath for 2 min. One-tenth ml of a solution containing 7.2 M urea, 1% sodium dodecyl sulfate, and 2% beta-mercaptoethanol was added, the preparation was sonicated for 30 Sec. and then boiled for another 2 min. Ten to forty microliters of this preparation were applied to a 10% sodium dodecyl sulfate-polyacrylamide gel and subjected to electrophoresis (Wong, et al., 1983). The proteins in the gels were transferred electrophoretically to nitrocellulose and were reacted with anti-crystal antibody and [¹²⁵I] Staphylacoccus aureus Protein A as described in Schnepf and Whiteley (1981).

Insect toxicity assays. Hatchling tobacco hornworm (Manduca sexta) caterpillars were obtained from Drs. J. Truman and L. Riddiford (Department of Zoology, University of Washington). Cell extracts were prepared by sonicating the cells from a 150 ml overnight culture in 2 ml of 0.1 sodium phosphate buffer, pH 7.4. Cell suspensions of the recombinant Escherichia coli strains from 20-50 ml of overnight culture were prepared in a 0.25 to 0.3 ml volume. The extracts (0.2 ml) or suspensions (50 microliter) were spread on an agar-based diet (2-4 ml/vial; 4.8 cm² area; See Schesser, et al., 1977) and allowed to dry. One neonate caterpillar was placed in each vial and mortality was determined after 5 days. Each recombinant strain was tested two to four times in quintuplicate. A positive toxicity score indicted that all 5 larvae died within 5 days whereas a negative score indicted normal growth and development.

Plasmid constructions. The source of crystal protein DNA was pES1 (ATCC 31995) or its Tn5 insertion derivatives. The strategy used to delineate the toxin-encoding portion of the gene was to construct a vector (pHES16) which contained the 5' portion of the gene and was not lethal to the larvae of the tobacco hornworm. In a second construction, an adjacent segment of the crystal protein gene (the HindIII E fragment) was added to extend pHES16 thereby creating a sequence (pHES19) which encoded a toxic peptide. To determine which portion of the HindIII-E fragment contained the 3' terminus of the toxic activity, subfragments of HindIII-E were added to extend pHES16. To determine the 5' margin of the toxic peptide, the lacZ alpha peptide was fused to the crystal protein gene at the 10th and 50th codons.

pHES16--a toxin analysis vector. Deletions were made in pBR322 to remove the PvuII and XmnI sites at positions 2065 and 2030 in pBR322 (Sutcliff, 1979) by digestion with Bal 31 nuclease followed by religation. The XhoI-HindIII fragment of pES1-B8 containing a portion of Tn5 and the 5' portion of the crystal protein gene with its transcriptional and translational start signals (crosshatched bar in FIGURE 3B) was purified by agarose gel electrophoresis and DE52 chromatography. This fragment was cloned into the HindIII and SalI sites of the deleted pBR322 to give pHES16 (FIGURE 3B). This vector was digested with HindIII and bacterial alkaline phosphatase and was then used to accept HindIII fragments which would extend the crystal protein gene in the 3' direction as indicated by the dashed lines in FIGURE 4D.

pHES19--extension of the pHES16 crystal protein gene by addition of the HindIII E fragment of pES1. The HindIII E fragment (FIGURE 3A) was purified and ligated into pHES16 at the HindIII site. The orientation of this fragment in pHES16 was determined by digestion with the appropriate restriction enzymes and by an analysis of antigenic peptides as assayed by immunoblotting.

pHES32, 23, 25, 33 and 30--extension of the pHES16 crystal protein gene to Tn5 insertion points in the HindIII-E fragment. Insertion mutants containing Tn5 (see Wong, et al. (1983); also see FIGURE 4C) in the HindIII-E fragment were first deleted by XhoI digestion and religation to remove DNA between the Tn5 segment nearest the 5' end of the crystal protein gene and the XhoI site in the HindIII-D fragment of pES1 as shown in FIGURE 4C. The resulting HindIII fragment containing the 5'-proximal portion of the HindIII-E fragment of pES1 was purified and cloned into the HindIII site of pHES16 (FIGURE 4D). The orientation of the cloned HindIII fragment was determined by XhoI-PstI digestion; the correct orientation yielded a XhoI-PstI fragment common to all of these plasmids. pHES31 contains the same insert as pHES32 but in the opposite orientation.

pHES34, 35, 36 and 38--extension of pHES16 crystal protein gene to sequenced endpoints. The PvuII C fragment of pES1 was cloned into the SmaI site of M13mp8 in an orientation which put the end nearest the 3' end of the crystal protein gene closest to the HindIII site of the polylinker system of the phage (FIGURE 4B). New HindIII fragments containing 5' proximal portions of the HindIII E fragment were produced by making deletions. Thus, the insert for pHES34 was generated by partial digestion with DNAse I in the presence of Mn⁺⁺, followed by digestion with SalI, filling in with DNA polymerase I and religation. The inserts used for pHES35 and 36 were produced by partial digestion with Sau3AI followed by digestion with BamHI, purification and religation. For pHES34 and 36, the positions of the endpoints of the deletions were determined by sequencing. The Sau3AI site of the pHES35 endpoint was inferred from restriction enzyme digestion and comparison with the known sequence. After insertion into pHES16, the orientations of the HindIII fragments were determined by PstI digestion: a fragment common to all three plasmids, extending from the site in the vector to the site remaining from the mp8 linker system, was indicative of the correct orientation.

pHES38 was produced by cloning XmnI fragment No. 5 (FIGURE 3A) into the SmaI site of M13mp8 in an orientation such that the end nearest the 3' terminus of the crystal protein was nearest the HindIII site of the mp8 polylinker sequence. The position of the XmnI site was determined by sequencing. The resulting HindIII fragment was inserted into the HindIII site of pHES16 and the orientation was determined as described above.

pHES37, 39 and 40--extension of the pHES16 crystal protein gene to well defined endpoints. The deletion used to construct pHES35 regenerated the BamHI site of the mp8 polylinker system and this was used to make several additional deletions (FIGURE 4E). pHES35 was digested with BclI (243 bases from the 5' proximal HindIII site of the HindIII E fragment) and BamHI and re-circularized by ligation to form pHES37. pHES39 was formed by digesting pHES35 partially with the EcoRI* activity (Polisky, et al., 1975) followed by complete digestion with BamHI and the DNA polymerase I-catalyzed fill-in reaction. Purified fragments of the correct size were re-circularized by ligation to form pHES39. pHES35 was digested partially with Sau3AI, completely with BamHI and the fragments of the appropriate size were purified and re-circularized by ligation to produce pHES40. The positions of the endpoints were determined by comparing the sizes of the resulting HindIII fragments with the known sequence.

pHES 41, 43 and 50--crystal protein genes under lac promoter control. The NdeI C fragment of pES1 (FIGURE 3A) was purified; pUC13Cm was digested with XbaI and the 5' extentions of both the insert and the vector were filled in using DNA polymerase I and deoxyribonucleotides. Following ligation, pHES41 was obtained as a transformant containing the NdeI C fragment under the control of the lac promoter. pHES41 was digested with both BclI and BamHI and was then re-circularized by ligation to form pHES43. pHES50 was constructed by a three-way ligation of pUC13Cm which had been digested with SalI and EcoRI, the purified SalI-BclI fragment of pHES41 which contained the 5' portion of the crystal protein gene, and the BamHI-EcoRI fragment of M13mp8CN3 which contained the final 74 codons and the transcriptional terminator of the crystal protein gene (FIGURE 5A). Phage M13mp8CN3 was constructed by cloning the PvuII-NdeI fragment containing the 3' end of the crystal protein gene into the SmaI site of M13mp8 following a DNA polymerase I-catalyzed fill-in reaction, in an orientation placing the crystal protein coding sequence closest to the BamHI site of the phage. The crystal protein gene reading frame at this BamHI site is the same as at the BclI site at codon 645. M13mp8CN3 was also used as a sequencing template for the 3' end of the crystal protein gene.

pHES44, 45, 48, 49, 51 and 52--fusions at the 10th or 50th codons. The alpha-peptide of lacZ in pUC8 was fused in the same reading frame to the crystal protein at the 10th (pHES 44, 48 and 51) or the 50th (pHES 45, 49 and 52) codons. Partial digestion of pES1 with XmnI was followed by complete digestion with XhoI and fragments of 5-5.3 Kb were purified. pUC8 was digested with BamHI, the 5' extensions were filled in with DNA polymerase I and deoxyribonucleotides and this preparation was then digested with SalI. The pUC8 and pES1-derived DNAs were then ligated (FIGURE 5B). Plasmid DNAs from several colonies which hybridized with a crystal protein gene-specific probe (Kronstad, et al. 1983) were screened using restriction enzymes to distinguish pHES44 from pHES45. Although both plasmids contained the internal EcoRI fragments of 580 and 732 bp from this crystal protein gene, additional smaller fragments were also found in pHES44 and pHES45, respectively; these fragments were from the crystal protein gene-pUC8 junction. The BamHI sites from the 10th codon fusions of this series (pHES44, 48 and 51) are regenerated, while the 50th codon fusions of this series (pHES45, 49 and 52) have lost the BamHI sites. pHES48 and 49 are 3'-end deletion mutants of pHES44 and 45, respectively (FIGURE 5B). The SmaI-BclI fragments of the latter plasmids, containing the 5' portion of the crystal protein gene, were cloned into the SmaI and BamHI sites of pUC8. The modified crystal protein polypeptides produced from these plasmids initiate at the lac translational start site and reenter the lac alpha-peptide sequence out of phase following the 645th codon of the crystal protein gene. Plasmids pHES51 and 52 were made by mixing the same SmaI-EcoRI fragments used for pHES 48 and 49 above with SmaI-EcoRI cut pUC13Cm and the EcoRI-BamHI fragment from M13mp8CN3 used to make pHES50 (FIGURE 5B). The fragments were ligated and transformed into JM83; white transformants growing on MacConkey agar were screened to determine if they contained plasmids with the predicted restriction enzyme digestion products. pHES51 and 52 contain a 19 amino acid long N-terminal coding sequence from beta-galactosidase and the pUC8 linker sequence fused to the crystal protein gene at the 10th and 50th codons, respectively, and are fused, in phase, to the lac alpha-peptide following the 645th codon of the crystal protein gene at the 3' end.

pHES46 and 47--10 and 50th condon fusions terminating in Tn5 inserts. These plasmids were formed in a manner analogous to that of pHES44 and 45 above except that the XmnI-XhoI partial digest fragment were from the Tn5 insertion deletion mutant pES1-B22-1 (FIGURE 5C). The Tn5 insertion mutant used (B22, FIGURE 4A) was the same as that used to make pHES33.

### EXAMPLE I

### DNA sequence of a crystal protein gene and the deduced amino acid sequence of its gene products.

The Bacillus thuringiensis crystal protein gene has been partially sequenced (Wong, et al., 1983). However, since more than this partial sequence was required to determine which portion of the crystal protein gene coded for the "toxin" portion of the crystal protein, the DNA sequence for the remainder of the gene was determined, partly with cloned restriction fragments from the restriction sites shown in FIGURE 1. The PuvII fragment from bases 1845 to 3831 in FIGURE 1B was sequenced primarily by the DNAse I deletion method of Hong (1982) (see numbered sites in FIGURE 1B) but come gaps in the coding strand sequence were filled in by obtaining deletions through partial Sau3AI digestion (asterisks in FIGURE 1B). The complete sequence was determined for both strands except for occasional anomalies of one to a few bases on one or the other strand. Since the coding sequence was thought to end 200-400 bases distal to the PuvII site at base 3831 (Wong, et al., 1983), the determinations were extended to base 4222 just beyond a sequence resembling an Escherichia coli transcription terminator (Rosenberg and Court, 1979). The latter region was verified by S1 nuclease mapping (discussed below) as the site of transcriptional termination.

As shown earlier (Wong, et al., 1983), the crystal protein gene is transcribed in Bacillus thuringiensis during sporulation from two adjacent start sites (Bt I and Bt II in FIGURE 2A). In recombinant Escherichia coli strains containing the cloned gone, transcription begins at a site (Ec in FIGURE 2A) located between Bt I and BtII and is independent of the phase of growth. The base sequences of the -10 and -35 regions of Bt I and Bt II differ significantly from the consensus sequences recognized by RNA polymerase from vegetative Bacillus subtilis and by Escherichia coli RNA polymerase. Klier, et al., (1978) reported the isolation or two modified forms of RNA polymerase from sporulating cultures of Bacillus thuringiensis; one of these polymerases was found to transcribe in vitro a crystal protein gene cloned from the chromosome of Bacillus thuringiensis subsp. thuringiensis (Klier, et al., 1983). Interestingly, the transcribed sequence, which is nearly identical to a HindIII/EcoRI fragment of the gene we have cloned (FIGURES 2A and 2B, bases 2100-2215), does not contain -10 and -35 sequences corresponding to the sequence upstream from either the BtI or BtII start sites.

The complete sequence of the Bacillus thuringiensis crystal protein gene is shown in FIGURES 2A and 2B. FIGURES 2A and 2B also show the amino acid sequence deduced from the only extended open reading frame in the DNA sequence. The open reading frame codes for 1176 amino acids specifying a polypeptide with a calculated molecular weight of 133,500. This value agrees well with molecular weights of 134,000 and 136,000, respectively, determined for the protoxins from Bacillus thuringiensis subsp. kurstaki (Bulla, et al., 1981) and subsp. thuringiensis (Huber, et al., 1981). The deduced amino acid composition is very similar to the chemically determined amino acid compositions of either whole crystals or the purified protoxin of Bacillus thuringiensis subsp. kurstaki (see Table 1).

### EXAMPLE II

The 5'-proximal portion of the crystal protein gene encodes a toxic peptide. Several investigators have proposed that the ca. Mᵣ=135,000 crystal protein is a protoxin which is proteolytically processed to yield smaller toxic fragments (Huber and Luthy, 1981; Bulla, et al., 1979; Lilley, et al., 1980 and Chestukhina, et al., 1982). In addition, our earlier immunoblot analysis of Tn5 insertion mutants of pES1 (see Wong, et al., 1983) revealed that a crystal protein antigen of ca. Mᵣ 68,000 was correlated with the 5'-proximal segment of the crystal protein gene. It was important, therefore, to determine whether this segment of the gene encoded a toxic peptide and, if so, to delineate the minimum portion of the gene which could produce such toxic fragments. To that end a series of pES1-type plasmids were constructed that contained insertions and deletions in the crystal protein gene. These plasmids were used to transform Escherichia coli host and then the resultant "crystal proteins" were analyzed for toxicity.

The plasmid used to study 3' end deletions of the crystal protein gene was pHES16 (FIGURE 3B). This plasmid consists of the XhoI-HindIII fragment of a Tn5 insertion mutant, pES1-B8 (crosshatched bar in FIGURE 3B), which includes 485 bp of Tn5 and a portion of the crystal protein gene containing the promoter and the first 565 codons, placed in a modified pBR322 (see FIGURE 3 and the Materials and Methods section, supra, for details of this and ensuing plasmid constructions). Escherichia coli strains carrying this plasmid produced a ca. Mᵣ 58,000 crystal protein antigen and were not toxic to caterpillars (FIGURE 6A, and lane 8 of FIGURE 6B).

To determine if extension of this crystal protein gene could restore toxicity, the HindIII-E fragment of pES1 (FIGURE 3A) was inserted into the HindIII site of pHES16. When the HindIII-E fragment was inserted in the proper orientation, the recombinant Escherichia coli strain bearing the resulting plasmid, pHES19, was toxic to caterpillars (FIGURE 6A). Escherichia coli cells carrying pHES19 produced several polypeptides which reacted with antibodies to the crystal protein, the most prominent of which were ca. Mᵣ=104,000 and 70,000 (lane 2 of FIGURE 6B).

In general, throughout these experiments, it was observed that when substantial amounts of crystal protein antigen were detected, an array of antigenic polypeptides was seen (e.g., lanes 1-6 of FIGURE 6B) whether the entire crystal protein (lane 1 of FIGURE 6B) or truncated derivatives (lanes 2-6 of FIGURE 6B) were synthesized. These polypeptides were distinct from smaller cross-reactive peptides produced by Escherichia coli carrying pBR322 (lane 10 of FIGURE 6B). We presume that the multiple peptides originating from the crystal protein gene were produced either cotranslationally or post-translationally by proteolysis in Escherichia coli.

### EXAMPLE III

Delineation of the 3' end of the toxin-encoding segment. The experiments outlined in Example II demonstrated that the 5'-proximal portion of the crystal protein gene encodes a toxic peptide and that the 3' end of the toxin-encoding portion was in the HindIII-E fragment. To determine the 3' end of this region more precisely, a number of deleted plasmids were constructed. See FIGURE 6A. The crystal protein encoding sequence in these plasmids terminated either in Tn5, where all reading frames close within 30 bases of the end of the transposon (boxes in FIGURE 6A; Auerswald, et al., 1981) or in pBR322, where all reading frames close within 2-17 codons (no boxes in FIGURE 6A; Sutcliff, 1979). FIGURE 6A also indicates the results of toxicity tests of extracts of these recombinant strains performed on the tobacco hornworm.

As indicated in FIGURE 6A, four of the deleted plasmids having the crystal protein gene terminated by Tn5 (pHES32, 23, 25 and 33) conferred toxicity to Escherichia coli while one (pHES30) did not. Of the nine deleted plasmids with crystal protein gene sequences terminating in pBR322, five conferred toxicity (pHES19, 34, 35, 36, 37) and four (pHES38, 39, 40 and 16) did not. Plasmid pHES38 contains 603 codons of the crystal protein gene, which is the longest non-toxic segment of the gene tested, while plasmid pHES37 contains the shortest toxic segment: 645 codons of the crystal protein gene, 5 codons from the M13mp8 linker sequence and 2 codons from pBR322.

Panels B and C of FIGURE 6 show an immunoblot assay of the polypeptides produced by recombinant strains containing these plasmids. As shown in lanes 2-6 of FIGURE 6B and lanes 2-5 of FIGURE 6C, deleted plasmids which directed synthesis of crystal protein fragments of 645 codons or longer (and made Escherichia coli toxic to caterpillars) synthesized crystal protein antigens of ca. Mᵣ 70,000 and an additional longer peptide which had a size roughly proportional to the length of the segment of the crystal protein gene beyond codon 645. Some additional fainter bands were also seen, as mentioned above. The full-length crystal protein as encoded by pES1-B8, is shown in lane 1 of FIGURE 6B and 6C for comparison. Escherichia coli strains containing deleted plasmids having less than 645 codons of the crystal protein gene (lanes 7-9 of FIGURE 6B and lanes 6-8 of FIGURE 6C) were not toxic to caterpillars and produced crystal protein antigens of ca. Mᵣ 58,000-60,000. The antigenic polypeptide in lane 9 of FIGURE 3B was produced from pHES31 which contains the HindIII insert of pHES32 in the opposite orientation; extracts of the strain carrying this plasmid were not toxic. In most cases (lanes 7-9 of FIGURE 6B and lanes 7 and 8 of FIGURE 6C), the ca. Mᵣ 58,000 polypeptide produced by the nontoxic strains was detected poorly by the immunoblotting analysis. This may be due to an increased susceptibility to proteolysis or might indicate the loss of a major antigenic determinant of the crystal protein.

### EXAMPLE IV

5' and 3' modifications of the crystal protein gene. Knowing that the 5'-proximal portion of the crystal protein gene encodes the toxic peptide and that the 3' end of the "toxin-encoding" gene segment is located between codons 603 and 645 (see FIGURE 2B) more plasmids containing altered crystal protein genes (under control of either the lac promoter, or the lac promoter plus the beta-galactosidase translational initiation site) were constructed to assess the effect of additional changes at the 5' and 3' ends of the gene on toxicity. These plasmids are diagrammed in FIGURE 7A; their construction is described in Materials and Methods section, supra.

For this study the 5' end modifications to the crystal protein gene were of two types, and were located at two places in the gene: 1) the first 10 codons of the crystal protein gene were replaced with 9 codons (pHES44, 46 and 48) or 19 codons (pHES51) of the beta-galactosidase and polylinker system of pUC8 and pUC13, respectively, or 2) the first 50 codons of the crystal protein gene were replaced by the first 9 (pHES45, 47 and 49) or 19 (pHES52) codons of the beta-galactosidase and polylinker system of pUC8 and pUC13, respectively. Plasmids pHES41, 43 and 50 were under the transcriptional control of the lac promoter but were under translational control of the crystal protein ribosome binding site and served as controls for the effects of the 5'-end modifications: 1) the crystal protein coding sequence, stop codon, and transcriptional terminator (pHES41, 44 and 45), 2) codon 645 of the crystal protein gene fused either in phase (pHES43) or out of phase (pHES48 and 49) with the reading frame of the subsequent portion of the lac alpha-peptide, 3) the Tn5 insertion site B22 (FIGURE 3B) in pES1 (pHES46 and 47), 4) codon 645 of the crystal protein gene re-connected in phase to the final 74 codons and the transcriptional terminator of the crystal protein gene (pHES50, 51 and 52).

Extracts of Escherichia coli containing these plasmids were tested for toxicity to hatchling caterpillars. Our results indicate that plasmids having the translational start site of the crystal protein gene or the beta-galactosidase fusion to the 10th codon of the crystal protein gene were toxic regardless of the modification to the 3' end of the coding sequence. The beta-galactosidase fusions to the 50th codon of the crystal protein gene were all non-toxic, irrespective of the 3'-terminal coding sequence.

### EXAMPLE V

### Effects of the 5'-and 3'-end alterations.

This example demonstrates that alterations of the crystal protein at the 5' end or at both ends affected production of the crystal protein antigen.

As shown in lanes 1-3 of FIGURE 7B, alterations to the 5' end of the whole crystal protein gene allowed production of a ca. Mᵣ 135,000 antigenic peptide. While the presence of the crystal protein N-terminus (pHES41, lane 1) allowed production of several minor apparent cleavage products in addition to the full-sized crystal protein, strains containing fusions at the 10th codon (pHES44, lane 2) and especially at the 50th codon (pHES45, lane 3) produced one (or more) prominent apparent cleavage product of ca. Mᵣ 110,000. Lanes 4-6 of FIGURE 7B show the crystal protein antigens produced by bacteria containing pHES43, 46 and 47, respectively. The proteins produced from these plasmids had sizes of ca. Mᵣ 77,000, 85,000 and 80,000, respectively, in accord with the size of the coding sequence present in these plasmids. In addition, lanes 4 and 5 (the crystal protein N-terminus and the 10th codon fusion, respectively) contained polypeptides in the ca. Mᵣ 70,000 range, while no polypeptide of this size was seen in lane 6 (the 50th codon fusion).

The results of an immunoblot assay of extracts of Escherichia coli carrying plasmids with additional 3'-end alterations following codon 645 of the crystal protein gene are shown in FIGURE 7C. As noted above, the natural N-terminus or the 10th codon fusion allowed processing of some of the crystal protein antigen to a ca. Mᵣ 70,000 form. Lanes 1-4 of FIGURE 7C show that this was true whether the 3' sequence following residue 645 was the beta-galactosidase alpha-peptide, either in phase (pHES43, lane 1) or out of phase (pHES48, lane 3) or the final 74 codons of the crystal protein gene (pHES50, lane 2; pHES51, lane 4). If the peptide synthesized in the strain containing the 50th codon fusion were processed when the 3'-end sequence was the out-of-phase lac alpha-peptide, it would be expected that lane 5 would show two bands, each ca. Mᵣ 4,000 smaller than the two bands of ca. Mᵣ 70,000 and 74,000 seen in lane 3. Only the larger of these two expected bands was detected. Lane 6 of FIGURE 7C shows that some cleavage of the 50th codon fusion protein to a ca. Mᵣ 70,000 peptide took place when the final 74 codons of the crystal protein gene were present following codon 645. FIGURE 7C also shows that when the final 74 codons of the crystal protein gene were fused in phase following codon 645, the same prominent peptide of ca. Mᵣ 50,000 was detected in extracts of strains having different 5' ends (lanes 2, 4 and 6 of FIGURE 7C), suggesting that sequences distal to residue 645 can influence the overall conformation of the altered polypeptide. Since this common cleavage product was found in extracts of a strain carrying the non-toxic plasmid pHES52, it seems likely that the ca. Mᵣ 50,000 peptide would be non-toxic.

The immunoblot analysis in FIGURES 6B, 6C and 7C indicates that when the ca. Mᵣ 70,000 crystal protein fragment was produced, the amount of antigen detected was affected by the sequences distal to the crystal protein coding sequences. As shown in lane 2 of FIGURE 6B and lanes 2-5 of FIGURE 6C, the amount of antigenic material synthesized by strains bearing plasmids with crystal protein sequences fused at the 3'-end to pBR322 was greater than when the crystal protein sequences terminated in Tn5 (lanes 3-6 of FIGURE 6B). It seems unlikely that this difference was due to the loss of a particularly immunogenic segment of the protoxin since both longer and shorter pBR322-terminated plasmids promoted the increased synthesis of the antigen when compared to the amount made when Tn5 was at the 3' end. Preliminary estimates of the rate of protein degradation in these strains, which were made by performing imnunoblot analyses on extracts of cultures at various times after protein synthesis was blocked by the addition of chloramphenicol, indicated that the rate of degradation of the altered crystal protein was not markedly different in strains having pBR322 or Tn5 sequences distal to the crystal protein gene coding sequences (data not shown). As shown in FIGURE 7C, more antigen was produced when the 3' end of the crystal protein gene was present (lanes 2, 4 and 6) than when transcription terminated in the pUC vectors (lanes 1, 3 and 5 of FIGURE 7C).

### Tables

**Table I**

| Amino Acid | Crystal* | Protoxin* | Deduced |
|---|---|---|---|
| Asx | 12.45+ | 13.31 | 12.0 |
| Thr | 6.46 | 5.99 | 6.3 |
| Ser | 7.78 | 6.49 | 7.3 |
| Glx | 12.09 | 11.98 | 12.0 |
| Pro | 3.48 | 5.32 | 5.4 |
| Gly | 7.45 | 6.82 | 6.8 |
| Ala | 5.46 | 5.32 | 5.4 |
| 1/2 Cys | 1.66 | 1.66 | 1.5 |
| Val | 6.95 | 7.32 | 6.9 |
| Met | 0.83 | 0.83 | 0.8 |
| Ile | 5.63 | 5.40 | 6.0 |
| Leu | 7.95 | 7.49 | 8.7 |
| Tyr | 4.14 | 3.83 | 4.4 |
| Phe | 3.97 | 4.83 | 4.6 |
| Lys | 2.65 | 2.5 | 2.8 |
| His | 1.66 | 2.16 | 1.9 |
| Arg | 7.95 | 7.65 | 6.4 |
| Trp | 1.16 | 1.0 | 1.5 |

| | | | |
|---|---|---|---|
| + - values in mole % | | | |
| * - taken from Bulla, et al., (1981). | | | |

### Conclusion

Thus it can be seen that the present invention discloses a toxin-encoding segment of a Bacillus thuringiensis crystal protein gene that is expressible in recombinant host organisms. The present invention further discloses that the "toxin" protein product produced by these transformed recombinant strains is toxic to lepidopteran insects. Since the toxin peptide is approximately half the size of the Bacillus thuringiensis crystal protein protoxin peptide, standard insecticidal preparations containing the protoxin crystals can be made to be twice as effective per given dose (or equally as effective at half the dose) by utilizing the smaller toxin fragment instead of the larger protoxin polypeptide.

Various modifications of the invention in addition to those shown and described herein will become apparent to those skilled in the art from the foregoing description and accompanying figures. Such modifications are intended to fall within the scope of the appended claims.

## Claims

1. A DNA fragment encoding a toxin portion of a Bacillus thuringiensis crystal protein peptide wherein said fragment is a toxin-encoding DNA sequence exhibiting sequence homology with the toxin-encoding DNA sequence beginning with base pair 527 and ending with base pair 2461 as shown in Figures 2A and 2B and wherein said sequence encodes a peptide having Bacillus thuringiensis crystal protein insecticidal activity.

2. A DNA fragment according to Claim 1 wherein the sequence coding for codons 1 to 10 may be replaced.

3. A DNA fragment according to Claim 1 or Claim 2 which also comprises DNA exhibiting sequence homology with a DNA sequence comprised of base pairs 4140 to 4185 (the transcriptional terminator) as shown in Figures 2A and 2B.

4. A recombinant microbial strain transformed by DNA according to any one of Claims 1 to 3 to express a polypeptide having the immunological properties of a toxin portion of the crystal protein of Bacillus thuringiensis.

5. A recombinant strain according to Claim 4 wherein said strain is a strain of bacteria or yeast.

6. A recombinant strain according to Claim 5 which is bacterial and selected from Escherichia coli, Bacillus subtilis, and bacteria that are endogenous to green plants.

7. A hybrid recombinant plasmid capable of replication in a bacterial host species, said plasmid containing expressible heterologous DNA incorporating a fragment according to any one of Claims 1 to 3 coding for a polypeptide which has the immunological and toxic properties of a toxin portion of crystal protein of Bacillus thuringiensis, said plasmid further including an expression mechanism for said heterologous DNA which is recognized by the host species' system.

8. A plasmid according to Claim 7 wherein the bacterial host is selected from Escherichia coli, and bacteria that are endogenous to green plants.

9. A method for producing a toxin portion of a Bacillus thuringiensis crystal protein peptide comprising utilizing a recombinant plasmid which contains a DNA fragment according to any one of Claims 1 to 3 to transform microbial host organisms.

10. A method for increasing the amount of toxin peptide produced by a recombinant strain comprising utilizing a recombinant plasmid containing a DNA fragment according to any one of Claims 1 to 3 to transform microbial host organisms.

11. The use of a DNA fragment according to any one of Claims 1 to 3 in the recombinant expression of a polypeptide having the immunological properties of a toxin portion of the crystal protein of Bacillus thuringiensis.

## Patentansprüche

1. DNA-Fragment, das einen Toxinanteil eines Bacillus thuringiensis-Kristallproteinpeptids codiert, wobei das Fragment eine Toxin-codierende DNA-Sequenz ist, die eine Sequenzhomologie mit der Toxin-codierenden DNA-Sequenz aufweist, die mit dem Basenpaar 527 beginnt und mit dem Basenpaar 2461 endet, wie in den Figuren 2A und 2B gezeigt, und wobei die Sequenz ein Peptid codiert, das Bacillus thuringiensis-Kristallprotein-insektizide Wirkung aufweist.

2. DNA-Fragment nach Anspruch 1, wobei die die Codons 1 bis 10 codierende Sequenz ersetzt werden kann.

3. DNA-Fragment nach Anspruch 1 oder 2, das weiterhin DNA umfaßt, die eine Sequenzhomologie mit einer DNA-Sequenz aufweist, die die Basenpaare 4140 bis 4185 (der Transkriptionsterminator), wie in den Figuren 2A und 2B gezeigt, umfaßt.

4. Rekombinanter mikrobieller Stamm, der mit einer DNA nach einem der Ansprüche 1 bis 3 transformiert ist, zur Expression eines Polypeptids mit den immunologischen Eigenschaften eines Toxinanteils des Kristallproteins von Bacillus thuringiensis.

5. Rekombinanter Stamm nach Anspruch 4, wobei der Stamm ein Bakterien- oder Hefestamm ist.

6. Rekombinanter Stamm nach Anspruch 5, nämlich ein Bakterienstamm ausgewählt aus Escherichia coli, Bacillus subtilis und Bakterien, die endogen in grünen Pflanzen vorkommen.

7. Hybrides rekombinantes Plasmid, das zur Replikation in einer bakteriellen Wirtsart befähigt ist, wobei das Plasmid exprimierbare heterologe DNA enthält, in die ein Fragment nach einem der Ansprüche 1 bis 3 eingebaut ist, welches ein Polypeptid mit den immunologischen und toxischen Eigenschaften eines Toxinanteils des Kristallproteins von Bacillus thuringiensis codiert, wobei das Plasmid weiterhin einen Expressionsmechanismus für die heterologe DNA umfaßt, der durch das System der Wirtsart erkannt wird.

8. Plasmid nach Anspruch 7, wobei der bakterielle Wirt ausgewählt ist aus Escherichia coli und Bakterien, die endogen in grünen Pflanzen vorkommen.

9. Verfahren zur Herstellung eines Toxinanteils eines Bacillus thuringiensis-Kristallproteinpeptids, umfassend die Verwendung eines rekombinanten Plasmids, das ein DNA-Fragment nach einem der Ansprüche 1 bis 3 enthält, zur Transformation von mikrobiellen Wirtsorganismen.

10. Verfahren zur Steigerung der Menge von Toxinpeptid, das durch einen rekombinanten Stamm produziert wird, umfassend die Verwendung eines rekombinanten Plasmids, das ein DNA-Fragment nach einem der Ansprüche 1 bis 3 enthält, zur Transformation von mikrobiellen Wirtsorganismen.

11. Verwendung eines DNA-Fragments nach einem der Ansprüche 1 bis 3 für die rekombinante Expression eines Polypeptids mit den immunologischen Eigenschaften eines Toxinanteils des Kristallproteins von Bacillus thuringiensis.

## Revendications

1. Fragment d'ADN codant pour une partie de la toxine d'un peptide de la protéine cristalline du Bacillus thuringiensis, où ledit fragment est une séquence d'ADN codant pour une toxine présentant une homologie de séquence avec la séquence d'ADN codant pour une toxine commençant par la paire de bases 527 et se terminant par la paire de bases 2461, comme le montrent les figures 2A et 2B et où ladite séquence code pour un peptide possèdant l'activité insecticide de la protéine cristalline de Bacillus thuringiensis.

2. Fragment d'ADN selon la revendication 1 dans lequel la séquence codant pour les codons 1 à 10 peut être remplacée.

3. Fragment d'ADN selon la revendication 1 ou 2 qui comprend également de l'ADN présentant une homologie de séquence avec une séquence d'ADN englogant les paires de bases 4140 à 4185 (le signal de terminaison de la transcription) comme le montrent les figures 2A et 2B.

4. Souche microbienne recombinante transformé par l'ADN selon l'une des revendication 1 à 3 afin d'exprimer un polypeptide possèdant les propriétés immunologiques d'une partie de la toxine de la protéine cristalline de Bacillus thuringiensis.

5. Souche recombinante selon la revendication 4, laquelle souche est une souche de bactérie ou de levure.

6. Souche recombinante selon la revendication 5 qui est une souche bactérienne choisie parmi Escherichia coli, Bacillus subtilis et les bactéries endogènes pour les plantes vertes.

7. Plasmide recombinant hybride capable de se répliquer dans un hôte bactérien, ledit plasmide contenant une molécule d'ADN hétérologue susceptible d'être exprimée comprenant un fragment selon l'une des revendications 1 à 3 codant pour un polypeptide possèdant les propriétés immunologiques et toxiques d'une partie de la toxine de la protéine cristalline de Bacillus thuringiensis, ledit plasmide comprenant en outre un mécanisme d'expression pour ledit ADN hétérologue qui est reconnu par le système de l'espèce hôte.

8. Plasmide selon la revendication 7 pour lequel l'hôte bactérien est choisie parmi Escherichia coli et les bactéries endogènes pour les plantes vertes.

9. Procédé pour le production d'une partie de la toxine d'un peptide de la protéine cristalline de Bacillus thuringiensis, comprenant l'utilisation d'un plasmide recombinant qui contient un fragment d'ADN selon l'une des revendications 1 à 3 pour transformer les organismes microbiens hôtes.

10. Procédé destiné à accroître la quantité de toxine peptidique produite par une souche recombinante, comprenant l'utilisation d'un plasmide recombinant contenant un fragment d'ADN selon l'une des revendications 1 à 3 pour transformer les organismes microbiens hôtes.

11. Utilisation d'un fragment d'ADN selon l'une des revendications 1 à 3 dans l'expression recombinante d'un polypeptide possédant les propriétés immunologiques d'une partie de la toxine de la protéine cristalline de Bacillus thurgiensis.
